**Europäisches Patentamt**

**European Patent Office**

**Office européen des brevets**

(19)

(11) Veröffentlichungsnummer: **0 009 464**
A1

(12) # EUROPÄISCHE PATENTANMELDUNG

(21) Anmeldenummer: **79810092.1**

(51) Int. Cl.³: **A 61 K 7/50**

(22) Anmeldetag: **12.09.79**

(30) Priorität: **15.09.78 CH 9695/78**

(43) Veröffentlichungstag der Anmeldung: **02.04.80**
Patentblatt 80/7

(84) Benannte Vertragsstaaten: **AT BE CH DE FR GB IT LU NL SE**

(71) Anmelder: **Verband Aargauischer Käserei- und Milchgenossenschaften, Helgenfeld 1595, CH-5034 Suhr (CH)**

(72) Erfinder: **Schmid, Alfons, Hubel 6, CH-5034 Suhr (CH)**
Erfinder: **Amrein, Roman, Holzacherweg 3b, CH-5033 Buchs (CH)**
Erfinder: **Meier, Christian, Brummelstrasse 3, Ch-5033 Buchs (CH)**

(74) Vertreter: **Schmid, Rudolf et al, c/o ISLER & SCHMID Patentanwaltsbureau Walchestrasse 23, CH-8006 Zürich (CH)**

(54) **Schaumbad-Präparat.**

(57) Die Verwendung von Molke in einem Schaumbad-Präparat führt zu hautfreundlichen Bädern mit weichem angenehmen Schaum, die die Haut ernähren. Diese Anwendung ermöglicht die nützliche Verwertung eines Molkerei-Nebenproduktes.

EP 0 009 464 A1

ACTORUM AG

- 1 -

**BESCHREIBUNG**

Verband aargauischer Käserei- und
Milchgenossenschaften,
CH-5034   S u h r   [Schweiz]

Schaumbad-Präparat

Gegenstand der vorliegenden Erfindung ist ein
Schaumbad-Präparat, welches Molke enthält.

Die bei der Käseherstellung als Nebenprodukt
anfallende Molke findet infolge ihrer biologisch
hochwertigen Zusammensetzung seit längerer Zeit Verwendung als Diät-Nährmittel. So werden in verschiedenen Kurorten Molkekuren durchgeführt. Auch Getränke
auf Molkegrundlage sind im Handel.

Molke ist reich an Milchzucker an verschiedenen Gliedern des Vitamin-B-Komplexes und an zahlreichen Aminosäuren. Ihr pH-Wert liegt auf der sauren

0009464

- 2 -

Seite, im allgemeinen zwischen 5 und 6, üblicherweise bei 5,6 bis 5,8.

Es wurde nun gefunden, dass sich die Molke aufgrund der genannten Eigenschaften ausgezeichnet als
Bestandteil eines Schaumbad-Präparates eignet. Das
erfindungsgemässe Schaumbad-Präparat, das neben den
üblichen Bestandteilen, wie waschaktive Stoffe, Emulgatoren, Konservierungsmittel, Parfums, Farbstoffen
und dergleichen, Molke in unkonzentrierter, konzentrierter (im allgemeinen 48 % Trockengehalt) oder
pulverisierter Form zugesetzt enthält, ergibt im
Wasser ein sehr hautfreundliches, milchiges Schaumbad mit einem pH-Wert, der demjenigen der menschlichen
Haut nahe liegt. Der Schaum ist weich und angenehm
und die Haut fühlt sich nach dem Bade weich und glatt,
aber weder ausgelaugt noch fettig, an. Auch empfindliche Hauttypen weisen keinerlei allergische oder
andere Reizwirkungen auf.

Vorzugsweise wird konzentrierte Molke mit einem
Trockensubstanzgehalt von 48 Gewichtsprozent verwendet, welche vor der Verarbeitung zum Schaumbad-Präparat mit Vorteil einer Sterilisation unterzogen wird.

Ausser der Molke enthält das Präparat die
üblichen Komponenten eines Schaumbadpräparates. Als
waschaktive Komponenten eignen sich z.B. die üblichen
oberflächenaktiven Verbindungen mit einer durchschnittlichen Kettenlänge von 12 Kohlenstoffatomen,
wie "Texapon N 40" (Henkel), "Steinapol" (REWO),

0009464

- 3 -

"Marlopon AT 50" (Hüls Chemie), und andere.

Als Emulgatoren eignen sich unter anderem folgende Handelsprodukte "Comperlan KD" (Henkel), "Steinamid" (REWO) und "Marlamid D 1885" (Hüls Chemie).

Im allgemeinen empfiehlt sich ein Zusatz von cirka 2 Gewichtprozent Konservierungsmittel, bezogen auf den Molkengehalt. Als Konservierungsmittel können z.B. "Tenox 2" (Eastman Kodak), "Bronidox L" (Henkel), Methylparaben, Propylparaben, Ascorbinsäure, usw. verwendet werden.

Beispiel

| Rezeptur | Gewichtsprozent |
|---|---|
| "Texapon N 40" (Henkel) | 50,00 |
| "Comperlan KD" (Emulgator) | 5,00 |
| Parfum IFF (FAC 25.567) | 3,00 |
| Molkenkonzentrat 48 % T.S. | 41,60 |
| Ascorbinsäure ) | 0,10 |
| "Tenox 2" } [Konservierungsmittel] | 0,10 |
| "Bronidox L" } | 0,20 |
| | 100,00 |
| pH | 5,6 |

Zur Herstellung des Schaumbad-Präparates wird folgende Apparatur verwendet : Rostfreier Stahlkessel, geschlossene Bauweise, mit stufenlosem Ankerrührwerk 0 bis 60 Umdrehungen pro Minute, Mantelheizung bzw. -kühlung und Temperaturmessung. Bodenauslauf mit versenktem Ventil.

"Comperlan KD" wird in den Rührkessen vorgelegt, auf 40°C erwärmt und das Parfum zugegeben. Nach 5 Minuten wird "Tenox 2" eingerührt. Sobald eine homogene Masse vorliegt, wird bei einer Temperatur von 30°C "Texapon N 40" und das Molkenkonzentrat zugemischt. Unter langsamem Rühren werden Ascorbinsäure und "Bronidox L" in der Mischung gelöst. Anschliessend wird noch während 15 Minuten gerühr unter Vermeidung von Lufteinschluss und das Präparat in beliebige Verpackung abgefüllt. Das Produkt ist stabil, ohne Ausfällung oder Phasentrennung und weist ein pH von 5,6 auf.

Zur Herstellung eines Schaumbades werden 0,075 bis 0,1 ml des Präparates pro Liter Wasser verwendet. Das pH des Bades beträgt etwa 7.

Verband aargauischer Käserei-
und Milchgenossenschaften,
CH-5034   S u h r     [Schweiz]

PATENTANSPRÜCHE:

1.     Schaumbad-Präparat, dadurch gekennzeichnet,
dass es zusätzlich zu den waschaktiven Stoffen Molke
enthält.

2.     Schaumbad-Präparat nach Patentanspruch 1,
dadurch gekennzeichnet, dass es 16 bis 22 Gewichtsprozent Molke, berechnet als Trockensubstanz, enthält.

3.     Schaumbad-Präparat nach Patentanspruch 1,
dadurch gekennzeichnet, dass es ausserdem Emulgatoren und/oder Konservierungsmittel und/oder Parfum und/oder Farbstoffe enthält.

| Kategorie | Kennzeichnung des Dokuments mit Angabe, soweit erforderlich, der maßgeblichen Teile | betrifft Anspruch |
|---|---|---|
| | **EINSCHLÄGIGE DOKUMENTE** | |
| X | DE - A - 1 949 207 (MILCHVERWERTUNG WESTERWALD) <br> * Ansprüche; Seite 2, Absatz 2 bis Seite 4, Absatz 2; das Beispiel * <br> .. <br> -- | 1,3 |
| X | LU - A - 42 250 (MOLKEREI-GENOSSEN-SCHAFT WESTERBURG) <br> * Ansprüche; Seite 3, Absatz 2 bis Seite 5, Absatz 3; das Beispiel * <br> .. <br> -- | 1,3 |
| X | FR - A - 1 359 693 (K. JANSEN) <br> * Zusammenfassung; Seite 1, Spalte 1, Absatz 3 bis Seite 2, Spalte 1, Absatz 2; Beispiel 2 * <br> .. <br> -- | 1-3 |
| X | FR - A - 820 846 (W. GERBER) <br> * Zusammenfassung; Seite 1, Zeilen 20-54; Seite 2, Zeilen 8-38; Seite 3, Zeilen 5-52 * <br> .... <br> ---- | 1,3 |

**KLASSIFIKATION DER ANMELDUNG (Int.Cl. 3)**

A 61 K 7/50

**RECHERCHIERTE SACHGEBIETE (Int. Cl. 3)**

A 61 K 7/50

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 20-12-1979 | VANHECKE |

EPA form 1503.1 06.78